(19) Europäisches Patentamt
European Patent Office
Office européen des brevets

(11) **EP 2 547 705 B1**

(12) **EUROPÄISCHE PATENTSCHRIFT**

(45) Veröffentlichungstag und Bekanntmachung des Hinweises auf die Patenterteilung:
**02.07.2014  Patentblatt 2014/27**

(21) Anmeldenummer: **11707684.4**

(22) Anmeldetag: **14.03.2011**

(51) Int Cl.:
*C08F 220/06* (2006.01)     *A61L 15/60* (2006.01)
*C08F 6/00* (2006.01)     *C08J 3/24* (2006.01)
*C08K 5/42* (2006.01)     *C08K 5/53* (2006.01)

(86) Internationale Anmeldenummer:
**PCT/EP2011/053764**

(87) Internationale Veröffentlichungsnummer:
**WO 2011/113777 (22.09.2011 Gazette 2011/38)**

(54) **VERFAHREN ZUR HERSTELLUNG WASSERABSORBIERENDER POLYMERPARTIKEL MIT VERBESSERTER FARBSTABILITÄT**

PROCESS FOR THE FABRICATION OF WATERABSORBING POLYMERIC PARTICLES HAVING IMPROVED COLOUR STABILITY

PROCÉDÉ DE FABRICATION DE PARTICULES ABSOBANTES AYANT UNE MEILLEURE STABILITÉ DE COULEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.03.2010  EP 10156480**

(43) Veröffentlichungstag der Anmeldung:
**23.01.2013  Patentblatt 2013/04**

(73) Patentinhaber: **BASF SE**
**67056 Ludwigshafen (DE)**

(72) Erfinder:
- **HERFERT, Norbert**
  **63674 Altenstadt (DE)**
- **DANIEL, Thomas**
  **67165 Waldsee (DE)**
- **PARCHANA, Bootsara**
  **Chonburi**
  **20230 (TH)**

(56) Entgegenhaltungen:
**WO-A1-2004/084962     WO-A2-2010/012762**
**US-B1- 6 359 049**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft Verfahren zur Herstellung wasserabsorbierender Polymerpartikel, wobei mindestens eine anorganische Phosphorsäure und/oder deren Salz und mindestens eine organische 2-Hydroxysäure und/oder deren Salz zugesetzt wird, wobei der Phosphor in der anorganischen Phosphorsäure eine Oxidationszahl von weniger als +V und die organische 2-Hydroxysäure keine ethylenisch ungesättigten Gruppen aufweist, sowie die gemäß dem erfindungsgemäßen Verfahren erhältlichen wasserabsorbierenden Polymerpartikel.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 $g/cm^2$ (AUL0.3psi) durchläuft ein Maximum.

Zur Verbesserung der Anwendungseigenschaften, wie beispielsweise Permeabilität des gequollenen Gelbetts (SFC) in der Windel und Absorption unter einem Druck von 49.2 $g/cm^2$ (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 $g/cm^2$ (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet, thermisch oberflächennachvernetzt und getrocknet. Dazu geeignete Vernetzer sind Verbindungen, die mit mindestens zwei Carboxylatgruppen der wasserabsorbierenden Polymerpartikel kovalente Bindungen bilden können.

Ein bei wasserabsorbierenden Polymerpartikeln oft auftretendes Problem sind Verfärbungen, die beim Lagern unter höherer Temperatur oder höherer Luftfeuchtigkeit auftreten. Derartige Bedingungen treten oft bei Lagerung in tropischen oder subtropischen Ländern auf. Unter solchen Bedingungen neigen wasserabsorbierende Polymerpartikel zum Vergilben, sie können sogar braune oder gar fast schwarze Färbung annehmen. Diese Verfärbung des eigentlich farblosen wasserabsorbierenden Polymerpartikeln ist unansehnlich und unerwünscht, da sie insbesondere bei den erwünschten dünnen Hygieneprodukten sichtbar ist und Verbraucher unansehnliche Hygieneprodukte ablehnen. Die Ursache für die Verfärbung ist nicht vollständig geklärt, allerdings scheinen reaktive Verbindungen wie Restmonomere aus der Polymerisation, die Verwendung mancher Initiatoren, Verunreinigungen des Monomeren oder des Neutralisationsmittels, Oberflächennachvernetzer oder Stabilisatoren der verwendeten Monomere eine Rolle zu spielen.

Gemäß WO 00/55245 A1 kann die Farbstabilität wasserabsorbierender Polymerpartikel durch Zusatz anorganischer Reduktionsmittel verbessert werden. Die anorganischen Reduktionsmittel können beispielsweise nach der Polymerisation dem Polymergel oder nach der thermischen Oberflächennachvernetzung zugesetzt werden.

WO 2006/058682 A1 lehrt, dass die Anwesenheit von Sauerstoff bei der thermischen Oberflächennachvernetzung zu Verfärbungen führt.

Gemäß WO 2004/084962 A1 wirkt sich die Verwendung von Sulfinsäuren als Polymerisationsinitiatoren günstig auf die Farbstabilität der erhaltenen wasserabsorbierenden Polymerpartikel aus.

WO 2008/092842 A1 und WO 2008/092843 A1 offenbaren die Beschichtung mit basischen Salzen zum selben Zweck.

Gemäß WO 2009/060062 A1 kann die Farbstabilität wasserabsorbierender Polymerpartikel mit Sulfonsäuren und deren Salzen erhöht werden, wobei die Sulfonsäuren bzw. deren Salze vorzugsweise unmittelbar vor der Oberflächennachvernetzung zugesetzt werden.

WO 03/014172 A2 beschreibt ein Verfahren zur Herstellung wasserabsorbierender Polymerpartikel, wobei die verwendete Acrylsäure vorher mit einem Aldehydfänger behandelt wurde, da insbesondere die Anwesenheit von Aldehyden zu Verfärbungen führen soll.

Aufgabe der vorliegenden Erfindung war die Bereitstellung eines Verfahrens zur Herstellung wasserabsorbierender Polymerpartikel mit verbesserter Farbstabilität. Gleichzeitig sollten die wasserabsorbierenden Polymerpartikel, insbesondere bei längerer Lagerung in warmer und feuchter Umgebung, keine unangenehmen Gerüche entwickeln.

Gelöst wurde die Aufgabe durch ein Verfahren zur Herstellung wasserabsorbierender Polymerpartikel durch Polymerisation einer Monomerlösung oder -suspension, enthaltend

a) mindestens ein ethylenisch ungesättigtes, säuregruppentragendes Monomer, das zumindest teilweise neutralisiert sein kann,
b) mindestens einen Vernetzer,
c) mindestens einen Initiator,

d) optional ein oder mehrere mit den unter a) genannten Monomeren copolymerisier-bare ethylenisch ungesättigte Monomere und

e) optional ein oder mehrere wasserlösliche Polymere,

umfassend die Schritte Polymerisation der Monomerlösung zu einem Polymergel i), optional Zerkleinerung des erhaltenen Polymergels ii),Trocknung des Polymergels iii), Mahlung und Klassierung des getrockneten Polymergels zu Polymerpartikeln iv), und optional thermischer Oberflächennachvernetzung der klassierten Polymerpartikel v), dadurch gekennzeichnet, dass vor, während oder nach einem der Schritte i) bis v) getrennt oder zusammen

- mindestens eine anorganische Phosphorsäure und/oder deren Salz und
- mindestens eine organische 2-Hydroxysäure und/oder deren Salz,

zugesetzt wird, wobei der Phosphor in der anorganischen Phosphorsäure eine Oxidationszahl von weniger als +V und die organische 2-Hydroxysäure keine ethylenisch ungesättigten Gruppen aufweist, und dass die organische 2-Hydroxysäure und/oder deren Salz vor Schritt i) zugesetzt wird.

Die anorganischen Phosphorsäuren und/oder deren Salze bzw. die organischen 2-Hydroxysäuren und/oder deren Salze werden vorzugsweise als wässrige Lösungen dosiert.

Geeignete anorganische Phosphorsäuren und/oder deren Salze sind unterphosphorige Säure, hypophosphorige Säure, Ammoniumphosphit, Ammoniumhypophosphit, Natriumphosphit, Natriumhypophosphit, Kaliumphosphit und Kaliumhypophosphit.

Ganz besonders bevorzugte anorganische Phosphorsäuren und/oder deren Salze sind hypophosphorige Säure und deren Natriumsalz.

Geeignete organische 2-Hydroxysäuren und/oder deren Salze sind 2-Hydroxy-2-sul-fonatoessigsäure, 2-Hydroxy-2-sulfonatopropionsäure, 2-Hydroxy-2-phosphonato-essigsäure, 2-Hydroxy-2-phosphonatopropionsäure, Hydroxyethyliden-1,1'-diphos-phonsäure, sowie deren Ammonium-, Natrium- und Kaliumsalze.

Ganz besonders bevorzugte organische 2-Hydroxysäuren und/oder deren Salze sind 2-Hydroxy-2-sulfonatoessigsäure, 2-Hydroxy-2-phosphonatoessigsäure und Hydroxy-ethyliden-1,1'-diphosphonsäure, sowie deren Natriumsalze.

Die Anwesenheit größerer Mengen an Reduktionsmittel, beispielsweise 2-Hydroxy-2-sulfinatoessigsäure und deren Salze, in Schritt i) wirkt sich nachteilig auf die Eigenschaften der wasserabsorbierenden Polymerpartikel aus, insbesondere auf die Zentrifugenretentionskapazität (CRC) und die Extrahierbaren. Daher werden in Schritt i) vorzugsweise weniger als 0,1 Gew.-%, besonders bevorzugt weniger als 0,02 Gew.-%, ganz besonders bevorzugt weniger als 0,01 Gew.-%, eines Reduktionsmittels, bezogen auf die wasserabsorbierenden Polymerpartikel, eingesetzt. Reduktionsmittel im Sinne der vorliegenden Erfindung sind Verbindungen mit Heteroatomen, wobei die Heteroatome nicht ihre maximale Oxidationszahl aufweisen. Sulfonsäuren und Phosphonsäuren sind also keine Reduktionsmittel im Sinne der vorliegenden Erfindung.

Vorzugsweise wird die anorganische Phosphorsäure und/oder deren Salz nach Schritt i) und vor Schritt iii) zugesetzt.

Die anorganischen Phosphorsäure und/oder deren Salze sind Reduktionsmittel und werden daher vorzugsweise erst nach beendeter Polymerisation zugesetzt. Werden der anorganischen Phosphorsäuren und/oder deren Salze als wässrige Lösung vor der Trocknung zugesetzt, so kann dass eingesetzte Lösungsmittel ohne zusätzlichen Schritt bei der Trocknung entfernt werden.

Die organischen 2-Hydroxysäure und/oder deren Salze werden optimal in die wasserabsorbierenden Polymerpartikel eingearbeitet, wenn sie in einem möglichst frühen Verfahrensschritt zugesetzt werden.

Die Einsatzmenge an anorganischer Phosphorsäure und/oder deren Salz, bezogen auf die wasserabsorbierenden Polymerpartikel, beträgt vorzugsweise von 0,001 bis 5 Gew.-%, besonders bevorzugt von 0,01 bis 2 Gew.-%, ganz besonders bevorzugt von 0,1 bis 1 Gew.-%.

Die Einsatzmenge an organischer 2-Hydroxysäuren und/oder deren Salz, bezogen auf die wasserabsorbierenden Polymerpartikel, beträgt vorzugsweise von 0,001 bis 5 Gew.-%, besonders bevorzugt von 0,01 bis 1,5 Gew.-%, ganz besonders bevorzugt von 0,05 bis 0,75 Gew.-%.

Der vorliegenden Erfindung liegt die Erkenntnis zugrunde, dass die erfindungsgemäß einzusetzenden anorganischen Phosphorsäuren und organischen 2-Hydroxysäuren synergistisch wirken.

Im Folgenden wird die Herstellung der wasserabsorbierenden Polymerpartikel näher erläutert:

Die wasserabsorbierenden Polymerpartikel werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispiels-weise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymemetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm$^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren.

Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffpero-xid/Natriumbisulfit. Vorzugsweise werden Mischungen aus thermischen Initiatoren und Redox-Initiatoren eingesetzt, wie Natriumperoxodisulfat/Wasserstoffperoxid/Ascor-binsäure. Als reduzierende Komponente wird aber vorzugsweise das Dinatriumsalz der 2-Hydroxy-2-sulfonatoessigsäure oder ein Gemisch aus dem Dinatriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Geignete Photoinitiatoren sind beispielsweise α-Spalter, H-abstrahierende Systeme und Azide. Geeignete α-Spalter bzw. H-abstrahierende Systeme sind beispielsweise Benzophenon-Derivate, wie Michlers-Keton, Phenanthren-Derivate, Fluoren-Derivate, Anthrachinon-Derivate, Thioxanton-Derivate, Cumarin-Derivate, Benzoinether und deren Derivate, Azostarter, wie die oben genannten Radikalbildner, substituierte Hexaarylbisimidazole oder Acylphosphinoxide. Geeignete Azide sind beispielsweise 2-(N,N-Dimethylamino)-ethyl-4-azidocinnamat, 2-(N,N-Dimethyl-amino)-ethyl-4-azidonaphthyl-keton, 2-(N,N-Dimethylamino)-ethyl-4-azidobenzoat, 5-Azido-1-naphthyl-2'-(N,N-dime-thylamino)ethylsulfon, N-(4-Sulfonylazidophenyl)maleinimid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclohexanon und 2,6-Bis-(p-azido-benzyliden)-4-methylcyclohexanon.

Mit den ethylenisch ungesättigten, säuregruppentragenden Monomeren a) copolymeri-sierbare ethylenisch ungesättigte Monomere d) sind beispielsweise Acrylamid, Methacrylamid, Hydroxyethylacrylat, Hydroxyethylme-thacrylat, Dimethylaminoethylmethacrylat, Dimethylaminoethylacrylat, Dimethylaminopropylacrylat, Diethylaminopropy-lacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisie-rung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

In Verfahrensschritt i) wird die Monomerlösung oder -suspension polymerisiert. Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt ii) zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation i) und Trocknung iii) zusammengefasst werden, wie in WO 2008/040715 A2 und WO 2008/052971 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkali-metallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

In Verfahrensschritt iii) wird das erhaltene Polymergel getrocknet. Die Trockner unterliegen keiner Beschränkung. Die Trocknung des Polymergels wird aber vorzugsweise mit einem Bandtrockner durchgeführt bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Moisture Content" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

In Verfahrensschritt iv) wird das getrocknete Polymergel gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA empfohlenen Testmethode Nr. WSP 220.2-05 "Partikel Size Distribution" ermittelt werden, wobei die Masseanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 µm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 µm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur Verbesserung der Eigenschaften in einem weiteren Verfahrensschritt v) thermisch oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle A-midoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydro-xyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetra-hydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyloxazolidin-2-on, Oxazolidin-2-on und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; DE), Hosokawa Bepex® Disc Dryer (Hosokawa Micron GmbH; Leingarten; DE) und Nara Paddle Dryer (NARA Machinery Europe; Frechen; DE). Überdies können auch Wirbelschichttrockner eingesetzt werden.

Die thermische Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Oberflächennachvernetzungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Ein weiterer Gegenstand der vorliegenden Erfindung sind die gemäß dem erfindungsgemäßen Verfahren erhältlichen wasserabsorbierenden Polymerpartikel.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA empfohlenen Testmethode Nr. WSP 241.2-05 "Centrifuge Retention Capacity" bestimmt.

Die gemäß dem erfindungsgemäßen Verfahren hergestellten wasserabsorbierenden Polymerpartikel weisen eine Absorption unter einem Druck von 49,2 $g/cm^2$ von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 $g/cm^2$ der wasserabsorbierenden Polymerpartikel beträgt üblicherweise weniger als 35 g/g. Die Absorption unter einem Druck von 49,2 $g/cm^2$ wird analog der von der EDANA empfohlenen Testmethode Nr. WSP 242.2-05 "Absorption under Pressure" bestimmt, wobei statt eines Drucks von 21,0 $g/cm^2$ ein Druck von 49,2 $g/cm^2$ eingestellt wird.

Die wasserabsorbierenden Polymerpartikel werden mittels der nachfolgend beschriebenen Testmethoden geprüft.

Die mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (Avenue Eugène Plasky 157, 1030 Brussels, Belgium, www.edana.org) und INDA (1100 Crescent Green, Cary, NC 27518, U.S.A., www.inda.org). Diese Veröffentlichung ist sowohl von EDANA als auch von INDA erhältlich.

Methoden:

Die Messungen sollten, wenn nicht anders angegeben, bei einer Umgebungstemperatur von 23 $\pm$ 2 °C und einer relativen Luftfeuchte von 50 $\pm$ 10 % durchgeführt werden. Die wasserabsorbierenden Polymerpartikel werden vor der Messung gut durchmischt.

Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Centrifuge Retention Capacity" bestimmt.

Absorption unter einem Druck von 49,2 $g/cm^2$ (Absorption under Pressure)

Die Absorption unter einem Druck von 49,2 $g/cm^2$ (AUL0.7psi) wird analog der von der EDANA empfohlenen Testmethode Nr. WSP 242.2-05 "Absorption under Pressure" bestimmt, wobei statt eines Drucks von 21,0 $g/cm^2$ (AUL0.3psi) ein Druck von 49,2 $g/cm^2$ (AUL0.7psi) eingestellt wird.

Extrahierbare

Die Extrahierbaren werden gemäß der von der EDANA empfohlenen Testmethode Nr. WSP 270.2-05 "Extractable" bestimmt.

Flüssigkeitsweiterleitung (Saline Flow Conductivity)

Die Flüssigkeitsweiterleitung (SFC) einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$SFC\ [cm^3s/g] = (Fg(t=0)xL0)/(dxAxWP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in $g/cm^3$, A die Fläche der Gelschicht in $cm^2$ und WP der hydrostatische Druck über der Gelschicht in $dyn/cm^2$.

Gelbettpermeabilität (Gel Bed Permeability)

Die Gelbettpermeabilität (GBP) einer gequollenen Gelschicht unter Druckbelastung von 0,3 psi (2070 Pa) wird, wie in US 2005/02567575 beschrieben (Absätze [0061] und [0075]), als Gel-Bed-Permeability einer gequollenen Gelschicht aus wasserabsorbierenden Polymerpartikeln bestimmt.

CIE-Farbzahl (L, a, b)

Die Farbmessung wird entsprechend dem CIELAB-Verfahren (Hunterlab, Band 8, Jahrgang 1996, Heft 7, Seiten 1 bis 4) mit einem Colorimeter, Modell "LabScan XE S/N LX17309" (HunterLab, Reston, US) durchgeführt. Dabei werden die Farben über die Koordinaten L, a und b eines dreidimensionalen Systems beschrieben. Dabei gibt L die Helligkeit an, wobei L = 0 schwarz und L = 100 weiß bedeutet. Die Werte für a und b geben die Position der Farbe auf den Farbachsen rot/grün bzw. gelb/blau an, wobei +a für rot, -a für grün, +b für gelb und -b für blau steht. Nach der Formel HC60 = L-3b wird der HC60-Wert berechnet.

Die Farbmessung entspricht dem Dreibereichsverfahren nach DIN 5033-6.

Alterungstest

Messung 1 (anfängliche Farbe): Eine Plastikschale mit 9 cm Innendurchmesser wird mit Superabsorberpartikeln überfüllt und diese dann mit einem Messer über den Rand glatt gestrichen und die CIE-Farbzahlen sowie der HC60-Wert bestimmt.

Messung 2 (nach Alterung): Eine Plastikschale mit 9 cm Innendurchmesser wird mit Superabsorberpartikeln überfüllt und diese dann mit einem Messer über den Rand glatt gestrichen. Die Schale wird dann offen in einen auf 60 °C temperierten Klimaschrank bei konstanter relativer Luftfeuchte von 86% gestellt. Die Schale wird nach Ablauf von 21 Tagen herausgenommen. Nach Abkühlen auf Raumtemperatur werden die CIE-Farbzahlen sowie der HC60-Wert erneut bestimmt.

Beispiele

Beispiel 1 (Vergleichsbeispiel)

In einem 2 l Edelstahlbecher wurden 326,7 g 50 Gew.-%ige Natronlauge und 849 g gefrorenes, entmineralisiertes Wasser vorgelegt. Unter Rühren wurden 392,0 g Acrylsäure zugegeben, wobei die die Geschwindigkeit der Zugabe so eingestellt wurde, dass die Temperatur 35°C nicht überstieg. Der Mischung wurde dann unter Rühren mit Hilfe eines Kühlbades abgekühlt. Als die Temperatur der Mischung auf 20°C abgefallen war, wurden 0,804 g dreifach ethoxyliertes Glycerintriacrylat, 0,041 g 2-Hydroxy-2-me-thyl-1-phenylpropan-1-on (DAROCUR®1173; Ciba Specialty Chemicals Inc.; Basel; CH) und 0,014 g 2,2-Dimethoxy-1,2-diphenylethan-1-on (IRGACURE® 651; Ciba Specialty Chemicals Inc.; Basel; CH) zugesetzt. Es wurde weiter abgekühlt, und bei Erreichen von 15°C wurde die Mischung durch Durchleiten von Stickstoff mittels einer Glasfritte von Sauerstoff befreit. Beim Erreichen von 0°C wurden 0,482 g Natriumpersulfat (gelöst in 5 ml entmineralisiertem Wasser) und 0,197 g 30 gew.-%ige Wasserstoffperoxid-Lösung (gelöst in 6 ml entmineralisiertem Wasser) zugesetzt und die Monomerlösung wurde in eine Glasschale überführt. Die Glasschale war so dimensioniert, dass sich eine Schichtdicke der Monomerlösung von 5 cm einstellte. Anschließend wurden 0,020 g Ascorbinsäure (gelöst in 5 ml entmineralisiertem Wasser) zugesetzt und die Monomerlösung mit Hilfe eines Glasstabes kurz durchgerührt. Die Glasschale mit der Monomerlösung wurde unter eine UV-Lampe gestellt (UV-Intensität = 20 mW/cm$^2$), wobei die Polymerisation einsetzte. Nach 16 Minuten wurde das erhaltene Polymergel dreimal mit Hilfe eines handelsüblichen Fleischwolfs mit 6 mm Lochscheibe extrudiert und im Umlufttrockenschrank bei 160°C eine Stunde lang getrocknet. Das getrocknete Polymergel wurde dann gemahlen und auf eine Partikelgröße von 150 bis 600 μm abgesiebt.

Dieses Grundpolymer wurde zur Oberflächennachvernetzung in einem Pflugschar®-Mischer Typ M5 mit Heizmantel (Gebr. Lödige Maschinenbau GmbH; Paderborn; DE) bei 23°C und einer Wellendrehzahl von 450 Umdrehungen pro Minute mittels einer Zweistoff-Sprühdüse mit einem Gemisch aus 1,0 Gew.-% 1,3-Propandiol, 0,06 Gew.-% N-(2-Hydroxyethyl)-2-oxazolidinon, 2,3 Gew.-% entmineralisiertem Wasser und 0,3 Gew.-% wässriger Aluminiumlaktat-Lösung (22 gew.-%ig), jeweils bezogen auf das Grundpolymer, beschichtet.

Nach dem Aufsprühen wurde die Produkttemperatur auf 170°C erhöht und das Reaktionsgemisch 60 Minuten lang bei dieser Temperatur und einer Wellendrehzahl von 60 Umdrehungen pro Minute gehalten. Das erhaltene Produkt wurde wieder auf Umgebungstemperatur abkühlen gelassen. Die oberflächennachvernetzten wasserabsorbierenden Polymerpartikel wurden auf eine Partikelgröße von 150 μm bis 600 μm abgesiebt und wiesen folgende Eigenschaften auf:

| | |
|---|---|
| CRC | = 37,4 g/g |
| AUL0.7psi | = 16,4 g/g |
| Extrahierbare | = 12,8 Gew.-% |

Die erhaltenen wasserabsorbierenden Polymerpartikel hatten eine CIE-Farbzahl von L = 88,7, a = -0,6 und b = 9,1, sowie einen HC60-Wert von 61,4.

Beispiel 2 (Vergleichsbeispiel)

Es wurde verfahren wie unter Beispiel 1. Der Monomerlösung wurden 0,392 g des Dinatriumsalzes der 2-Hydroxy-2-sulfonatoessigsäure (gelöst in 10 ml entmineralisiertem Wasser) statt der 0,020 g Ascorbinsäure zugesetzt.

Die oberflächennachvernetzten wasserabsorbierenden Polymerpartikel wurden auf eine Partikelgröße von 150 μm bis 600 μm abgesiebt und wiesen folgende Eigenschaften auf:

| | |
|---|---|
| CRC | = 37,7 g/g |
| AUL0.7psi | = 17,1 g/g |
| Extrahierbare | = 13,4 Gew.-% |

Die erhaltenen wasserabsorbierenden Polymerpartikel hatten eine CIE-Farbzahl von L = 90,0, a = -0,8 und b = 8,5, sowie einen HC60-Wert von 64,5.

Beispiel 3 (Vergleichsbeispiel)

Es wurde verfahren wie unter Beispiel 1. Der Monomerlösung wurden 0,980 g des Dinatriumsalzes der 2-Hydroxy-2-sulfonatoessigsäure (gelöst in 15 ml entmineralisiertem Wasser) statt der 0,020 g Ascorbinsäure zugesetzt.

Die oberflächennachvernetzten wasserabsorbierenden Polymerpartikel wurden auf eine Partikelgröße von 150 μm bis 600 μm abgesiebt und wiesen folgende Eigenschaften auf:

| | |
|---|---|
| CRC | = 37,9 g/g |
| AUL0.7psi | = 16,8 g/g |
| Extrahierbare | = 13,9 Gew.-% |

Die erhaltenen wasserabsorbierenden Polymerpartikel hatten eine CIE-Farbzahl von L = 90,3, a = -0,8 und b = 7,9, sowie einen HC60-Wert von 66,6.

Beispiel 4

Es wurde verfahren wie unter Beispiel 1. Der Monomerlösung wurden 0,392 g des Dinatriumsalzes der 2-Hydroxy-2-sulfonatoessigsäure (gelöst in 10 ml entmineralisiertem Wasser) statt der 0,020 g Ascorbinsäure zugesetzt. Zusätzlich wurde das dreimal extrudierte Polymergel mit 1,96 g Natriumhypophosphit (gelöst in 10 ml entmineralisiertem Wasser) versetzt und weitere zweimal extrudiert.
Die oberflächennachvernetzten wasserabsorbierenden Polymerpartikel wurden auf eine Partikelgröße von 150 $\mu$m bis 600 $\mu$m abgesiebt und wiesen folgende Eigenschaften auf:

| | |
|---|---|
| CRC | = 37,5 g/g |
| AUL0.7psi | = 16,6 g/g |
| Extrahierbare | = 13,6 Gew.-% |

Die erhaltenen wasserabsorbierenden Polymerpartikel hatten eine CIE-Farbzahl von L = 90,5, a = -0,8 und b = 6,9, sowie einen HC60-Wert von 69,8.

Beispiel 5

Es wurde verfahren wie unter Beispiel 1. Der Monomerlösung wurden 0,392 g des Dinatriumsalzes der 2-Hydroxy-2-sulfonatoessigsäure (gelöst in 10 ml entmineralisiertem Wasser) statt der 0,020 g Ascorbinsäure zugesetzt. Zusätzlich wurde das dreimal extrudierte Polymergel mit 3,92 g Natriumhypophosphit (gelöst in 15 ml entmineralisiertem Wasser) versetzt und weitere zweimal extrudiert.
Die oberflächennachvernetzten wasserabsorbierenden Polymerpartikel wurden auf eine Partikelgröße von 150 $\mu$m bis 600 $\mu$m abgesiebt und wiesen folgende Eigenschaften auf:

| | |
|---|---|
| CRC | = 37,8 g/g |
| AUL0.7psi | = 16,9 g/g |
| Extrahierbare | = 13,8 Gew.-% |

Die erhaltenen wasserabsorbierenden Polymerpartikel hatten eine CIE-Farbzahl von L = 91,1, a = -0,8 und b = 6,3, sowie einen HC60-Wert von 72,2.

Beispiel 6 (Vergleichsbeispiel)

Es wurde verfahren wie unter Beispiel 1. Zusätzlich wurde das dreimal extrudierte Polymergel mit 3,92 g Natriumhypophosphit (gelöst in 15 ml entmineralisiertem Wasser) versetzt und weitere zweimal extrudiert.
Die oberflächennachvernetzten wasserabsorbierenden Polymerpartikel wurden auf eine Partikelgröße von 150 $\mu$m bis 600 $\mu$m abgesiebt und wiesen folgende Eigenschaften auf:

| | |
|---|---|
| CRC | = 37,9 g/g |
| AUL0.7psi | = 16,2 g/g |
| Extrahierbare | = 13,5 Gew.-% |

Die erhaltenen wasserabsorbierenden Polymerpartikel hatten eine CIE-Farbzahl von L = 89,1, a = -0,7 und b = 8,2, sowie einen HC60-Wert von 74,5.

Beispiel 7

Es wurde verfahren wie unter Beispiel 1. Der Monomerlösung wurden 0,392 g Brüggolite® FF6 (gelöst in 10 ml entmineralisiertem Wasser) statt der 0,020 g Ascorbinsäure zugesetzt. Brüggolite® FF6 ist ein Gemisch aus dem Natriumsalz der 2-Hydroxy-2-sulfinatoessigsäure, dem Dinatriumsalz der 2-Hydroxy-2-sulfonatoessigsäure und Natriumbisulfit (Brüggemann Chemicals; Heilbronn; DE). Zusätzlich wurde das dreimal extrudierte Polymergel mit 1,96 g Natriumhypophosphit (gelöst in 10 ml entmineralisiertem Wasser) versetzt und weitere zweimal extrudiert.
Die oberflächennachvernetzten wasserabsorbierenden Polymerpartikel wurden auf eine Partikelgröße von 150 $\mu$m bis 600 $\mu$m abgesiebt und wiesen folgende Eigenschaften auf:

| CRC | = 43,9 g/g |
| AUL0.7psi | = 11,7 g/g |
| Extrahierbare | = 28,9 Gew.-% |

Die erhaltenen wasserabsorbierenden Polymerpartikel hatten eine CIE-Farbzahl von L = 90,1, a = -0,7 und b = 7,4, sowie einen HC60-Wert von 67,9.

Beispiel 8

Es wurde verfahren wie unter Beispiel 1. Der Monomerlösung wurden 0,980 g 2-Hydroxy-2-phosphonatoessigsäure (gelöst in 15 ml entmineralisiertem Wasser) statt der 0,020 g Ascorbinsäure zugesetzt. Zusätzlich wurde das dreimal extrudierte Polymergel mit 1,96 g Natriumhypophosphit (gelöst in 10 ml entmineralisiertem Wasser) versetzt und weitere zweimal extrudiert.

Die oberflächennachvernetzten wasserabsorbierenden Polymerpartikel wurden auf eine Partikelgröße von 150 $\mu$m bis 600 $\mu$m abgesiebt und wiesen folgende Eigenschaften auf:

| CRC | = 37,4 g/g |
| AUL0.7psi | = 17,3 g/g |
| Extrahierbare | = 12,8 Gew.-% |

Die erhaltenen wasserabsorbierenden Polymerpartikel hatten eine CIE-Farbzahl von L = 90,7, a = -0,8 und b = 6,8, sowie einen HC60-Wert von 70,3.

Beispiel 9

Es wurde verfahren wie unter Beispiel 1. Der Monomerlösung wurden 1,57 g 1-Hydroxy-1,1'-ethylidendiphosphonsäure (gelöst in 20 ml entmineralisiertem Wasser) statt der 0,020 g Ascorbinsäure zugesetzt. Zusätzlich wurde das dreimal extrudierte Polymergel mit 2,65 g Natriumhypophosphit (gelöst in 10 ml entmineralisiertem Wasser) versetzt und weitere zweimal extrudiert.

Die oberflächennachvernetzten wasserabsorbierenden Polymerpartikel wurden auf eine Partikelgröße von 150 $\mu$m bis 600 $\mu$m abgesiebt und wiesen folgende Eigenschaften auf:

| CRC | = 38,1 g/g |
| AUL0.7psi | = 16,5 g/g |
| Extrahierbare | = 14,0 Gew.-% |

Die erhaltenen wasserabsorbierenden Polymerpartikel hatten eine CIE-Farbzahl von L = 91,0, a = -0,9 und b = 6,2, sowie einen HC60-Wert von 72,4.

Beispiel 10 (Vergleichsbeispiel)

Es wurde verfahren wie unter Beispiel 1. Zusätzlich wurde das dreimal extrudierte Polymergel mit 3,14 g Natriumhypophosphit (gelöst in 10 ml entmineralisiertem Wasser) und 1,18g 2-Hydroxy-2-phosphonatoessigsäure (gelöst in 10 ml entmineralisiertem Wasser) versetzt und weitere zweimal extrudiert.

Die oberflächennachvernetzten wasserabsorbierenden Polymerpartikel wurden auf eine Partikelgröße von 150 $\mu$m bis 600 $\mu$m abgesiebt und wiesen folgende Eigenschaften auf:

| CRC | = 37,9 g/g |
| AUL0.7psi | = 17,4 g/g |
| Extrahierbare | = 13,0 Gew.-% |

Die erhaltenen wasserabsorbierenden Polymerpartikel hatten eine CIE-Farbzahl von L = 90,7, a = -0,7 und b = 7,1, sowie einen HC60-Wert von 69,4.

Beispiel 11 (Vergleichsbeispiel)

Es wurde verfahren wie unter Beispiel 1. Der Monomerlösung wurden 0,980 g Natriumhypophosphit (gelöst in 5 ml entmineralisiertem Wasser) statt der 0,020 g Ascorbinsäure zugesetzt. Zusätzlich wurde das dreimal extrudierte Polymergel mit 1,18 g 2-Hy-droxy-2-phosphonatoessigsäure (gelöst in 10 ml entmineralisiertem Wasser) versetzt und weitere zweimal extrudiert.

Die oberflächennachvernetzten wasserabsorbierenden Polymerpartikel wurden auf eine Partikelgröße von 150 $\mu$m bis 600 $\mu$m abgesiebt und wiesen folgende Eigenschaften auf:

| | |
|---|---|
| CRC | = 57,3 g/g |
| AUL0.7psi | = 10,2 g/g |
| Extrahierbare | = 46,8 Gew.-% |

Die erhaltenen wasserabsorbierenden Polymerpartikel hatten eine CIE-Farbzahl von L = 89,6, a = -0,8 und b = 8,0, sowie einen HC60-Wert von 65,4.

Beispiel 12

In einen Pflugschar®-Schaufeltrockner Typ VT 5R-MK mit 5 l Volumen (Gebr. Lödige Maschinenbau GmbH; Paderborn: DE) wurden 468 g entmineralisiertes Wasser, 244,3 g Acrylsäure, 1924,9 wässrige Natriumacrylat-Lösung (37,3 gew.-%ig) und 3,28 g Sartomer ® SR-344 (Diacrylat eines Polyethylenglykols mit einem Molgewicht von ca. 400 g/mol), vorgelegt und unter Durchperlen von Stickstoff 20 Minuten inertisiert. Die Welle des Reaktors wurde durchgehend mit 96 Umdrehungen pro Minute gedreht. Die Reaktionsmischung wurde dabei von außen so gekühlt, dass die nachfolgende Initiatorzugabe bei ca. 20°C erfolgte. Schließlich wurden in den Pflugschar®-Schaufeltrockner unter Rühren noch 2,139 g Natriumpersulfat (gelöst in 12,12 g entmineralisiertem Wasser), 1,19 g des Dinatriumsalzes der 2-Hydroxy-2-sulfonatoessig-säure (gelöst in 20 ml entmineralisiertem Wasser) und 0,127 g 30gew.-%ige wässrige Wasserstoffperoxid-Lösung (verdünnt mit 1,15 g entmineralisiertem Wasser) rasch hintereinander hinzugefügt. Die Reaktion setzte zügig ein und bei Erreichen einer Innentemperatur von 30°C wurde der Mantel mit 80°C heißem Wärmeträgermedium beheizt, um die Reaktion möglichst adiabat zu Ende zu führen. Nach Erreichen der Maximaltemperatur wurde wiederum gekühlt (Kühlflüssigkeit mit -12°C), so das entstandene Polymergel auf unter 50°C herabgekühlt und dann ausgetragen wurde. Das erhaltene Polymergel wurde dreimal mit Hilfe eines handelsüblichen Fleischwolfs mit 6 mm Lochscheibe extrudiert, mit 3,97 g Natriumhypophosphit (gelöst in 15 ml entmineralisiertem Wasser) versetzt und weitere zweimal extrudiert. Das Polymergel wurde dann im Umlufttrockenschrank bei 160°C eine Stunde lang getrocknet. Das getrocknete Polymergel wurde dann gemahlen und auf eine Partikelgröße von 150 bis 710 $\mu$m abgesiebt.

Dieses Grundpolymer wurde zur Oberflächennachvernetzung in einem Pflugschar®-Mischer Typ M5 mit Heizmantel (Gebr. Lödige Maschinenbau GmbH; Paderborn; DE) bei 23°C und einer Wellendrehzahl von 450 Umdrehungen pro Minute mittels einer Zweistoff-Sprühdüse mit einem Gemisch aus 1,0 Gew.-% 1,2-Propandiol, 0,125 Gew.-% N-(2-Hydroxyethyl)-2-oxazolidinon, 1,5 Gew.-% entmineralisiertem Wasser, 0,003 Gew.-% Span® 20 (Sorbitanmonolaurat) und 2,8 Gew.-% wässriger Aluminiumsulfat-Lösung (26,8 gew.-%ig), jeweils bezogen auf das Grundpolymer, beschichtet.

Nach dem Aufsprühen wurde die Produkttemperatur auf 180°C erhöht und das Reaktionsgemisch 50 Minuten lang bei dieser Temperatur und einer Wellendrehzahl von 60 Umdrehungen pro Minute gehalten. Das erhaltene Produkt wurde wieder auf Umgebungstemperatur abkühlen gelassen. Die oberflächennachvernetzten wasserabsorbierenden Polymerpartikel wurden auf eine Partikelgröße von 150 $\mu$m bis 710 $\mu$m abgesiebt und wiesen folgende Eigenschaften auf:

| | |
|---|---|
| CRC | = 27,5 g/g |
| AUL0.7psi | = 22,6 g/g |
| SFC | = 145 x $10^{-7}$ cm$^3$s/g |
| GBP | = 120 Darcies |

Die erhaltenen wasserabsorbierenden Polymerpartikel hatten eine CIE-Farbzahl von L = 92,5, a = -0,5 und b = 3,6, sowie einen HC60-Wert von 81,7.

Beispiel 13 (Vergleichsbeispiel)

Es wurde verfahren wie unter Beispiel 12. Der Monomerlösung wurden 0,020 g Ascorbinsäure (gelöst in 10 ml entmineralisiertem Wasser) statt des Dinatriumsalzes der 2-Hydroxy-2-sulfonatoessigsäure zugesetzt. Zusätzlich wurde das dreimal extrudierte Polymergel mit 4,77 g Natriumhypophosphit (gelöst in 15 ml entmineralisiertem Wasser) und 4,77 g des Dinatriumsalzes der 1-Hydroxy-1,1'-ethylidendiphosphonsäure (gelöst in 30 ml entmineralisiertem Wasser) statt mit 3,97 g Natriumhypophosphit versetzt und weitere zweimal extrudiert.

Die oberflächennachvernetzten wasserabsorbierenden Polymerpartikel wurden auf eine Partikelgröße von 150 μm bis 710 μm abgesiebt und wiesen folgende Eigenschaften auf:

| | |
|---|---|
| CRC | = 28,1 g/g |
| AUL0.7psi | = 23,8 g/g |
| SFC | = 138 x $10^{-7}$ cm$^3$s/g |
| GBP | = 12 Darcies |

Die erhaltenen wasserabsorbierenden Polymerpartikel hatten eine CIE-Farbzahl von L = 92,8, a = -0,8 und b = 4,0, sowie einen HC60-Wert von 80,8.

Beispiel 14

14,3 kg wässrige Natriumacrylat-Lösung (37,5 gew.-%ig), 1,4 kg Acrylsäure und 350 g entmineralisiertes Wasser wurden mit 10,6 g dreifach ethoxyliertem Glycerintriacrylat gemischt. Diese Lösung wurde in einem erwärmten, mit Stickstoffatmosphäre gefüllten Vertropfungsturm (180°C, 12 m Höhe, 2 m Durchmesser, Gasgeschwindigkeit 0,1 m/s im Gleichstrom, Vertropfer mit 40 mm Durchmesser, 2 mm Innenhöhe und einer Vertropferplatte mit 60 Bohrungen von je 200 μm Durchmesser) in einer Dosiergeschwindigkeit von 32 kg/h vertropft. Die Temperatur der Lösung betrug 25°C. Kurz vor dem Vertropfer wurde die Monomerlösung über einen statischen Mischer mit drei Lösungen gemischt. Als Lösung 1 wurde eine 6 gew.-%ige Lösung von 2,2'-Azobis-[2-(2-imidazo-lin-2-yl)-propan]-dihydrochlorid in entmineralisiertem Wasser, als Lösung 2 eine 6 gew.-%ige Lösung von Natriumperoxodisulfat in entmineralisiertem Wasser und als Lösung 3 eine 10 gew.-%ige Lösung von 2-Hydroxy-2-phosphonatoessigsäure in entmineralisiertem Wasser verwendet. Die Dosiergeschwindigkeit der Lösung 1 betrug 0,642 kg/h, die Dosiergeschwindigkeit der Lösung 2 betrug 0,458 kg/h und die Dosiergeschwindigkeit der Lösung 3 betrug 0,275 kg/h. Die erhaltenen Polymerpartikel wurden auf eine Partikelgröße von 150 bis 850 μm abgesiebt, um eventuell gebildete Agglomerate abzutrennen.

Dieses Grundpolymer wurde in einem Pflugschar®-Mischer Typ M5 mit Heizmantel (Gebr. Lödige Maschinenbau GmbH; Paderborn; DE) bei 23°C und einer Wellendrehzahl von 250 Umdrehungen pro Minute mittels einer Zweistoff-Sprühdüse mit 2,0 Gew.-% einer 30gew.-%igen wässrigen Natriumhypophosphit-Lösung, bezogen auf das Grundpolymer, beschichtet.

Nach dem Aufsprühen wurde das Reaktionsgemisch 15 Minuten lang bei einer Wellendrehzahl von 60 Umdrehungen pro Minute gehalten. Die beschichteten wasserabsorbierenden Polymerpartikel wurden auf eine Partikelgröße von 150 μm bis 850 μm abgesiebt und wiesen folgende Eigenschaften auf:

| | |
|---|---|
| CRC | = 32,7 g/g |
| AUL0.7psi | = 23,2 g/g |
| SFC | = 20 x $10^{-7}$ cm$^3$s/g |

Die erhaltenen wasserabsorbierenden Polymerpartikel hatten eine CIE-Farbzahl von L = 93,4, a = 0,3 und b = 2,4, sowie einen HC60-Wert von 86,2.

Beispiel 15

Es wurde verfahren wie unter Beispiel 14. Als Lösung 3 wurde eine 7 gew.-%ige Lösung von des Dinatriumsalzes der 2-Hydroxy-2-sulfonatoessigsäure in entmineralisiertem Wasser verwendet. Die Dosiergeschwindigkeit der Lösung 1 wurde auf 0,275 kg/h geändert und die Dosiergeschwindigkeit der Lösung 3 betrug 0,314 kg/h.

Das erhaltene Grundpolymer wurde zur Oberflächennachvernetzung in einem Pflugschar®-Mischer Typ M5 mit Heizmantel (Gebr. Lödige Maschinenbau GmbH; Paderborn; DE) bei 23°C und einer Wellendrehzahl von 450 Umdrehungen pro Minute mittels einer Zweistoff-Sprühdüse mit einem Gemisch aus 1,0 Gew.-% 1,2-Propandiol, 0,1 Gew.-% Ethylenglykoldiglycidylether, 1,0 Gew.-% entmineralisiertem Wasser, und 2,0 Gew.-% wässriger Natriumhypophosphit-Lösung (30 gew.-%ig), jeweils bezogen auf das Grundpolymer, beschichtet.

Nach dem Aufsprühen wurde die Produkttemperatur auf 150°C erhöht und das Reaktionsgemisch 60 Minuten lang bei dieser Temperatur und einer Wellendrehzahl von 60 Umdrehungen pro Minute gehalten. Das erhaltene Produkt wurde wieder auf Umgebungstemperatur abkühlen gelassen. Die oberflächennachvernetzten wasserabsorbierenden Polymerpartikel wurden auf eine Partikelgröße von 150 μm bis 850 μm abgesiebt.

150 g der oberflächenachvernetzte Polymerpartikel wurden in einer 500ml Polyethylenflasche mit 0,30 g Sipernat® D17 (hydrophobe Fällungskieselsäure) versetzt und mittels eines Taumelmischers vom Typ T2C (Willy A. Bachofen AG Maschinenfabrik; Basel; CH) 15 Minuten innig vermischt.

Die beschichteten wasserabsorbierenden Polymerpartikel wiesen folgende Eigenschaften auf:

| | |
|---|---|
| CRC | = 28,2 g/g |
| AUL0.7psi | = 22,0 g/g |
| SFC | = 31 x $10^{-7}$ cm$^3$s/g |

Die erhaltenen wasserabsorbierenden Polymerpartikel hatten eine CIE-Farbzahl von L = 93,2, a = 0,4 und b = 2,5, sowie einen HC60-Wert von 85,7.

Tab. 1: Ergebnisse nach dem Alterungstest

| Beispiel | L | a | b | HC 60 |
|---|---|---|---|---|
| 1*) | 59,6 | 6,5 | 14,8 | 15,2 |
| 2*) | 80,2 | 1,1 | 13,6 | 39,4 |
| 3*) | 81,4 | 1,0 | 13,4 | 41,2 |
| 4 | 83,2 | 0,1 | 7,8 | 59,8 |
| 5 | 83,8 | - 0,1 | 7,3 | 61,9 |
| 6*) | 77,3 | 4,9 | 12,1 | 41,0 |
| 7 | 82,8 | 0,4 | 9,0 | 55,8 |
| 8 | 83,6 | 0,3 | 7,7 | 60,5 |
| 9 | 84,2 | - 0,2 | 7,0 | 63,2 |
| 10*) | 83,0 | 0,4 | 8,1 | 58,7 |
| 11*) | 82,5 | 0,7 | 8,5 | 57,0 |
| 12 | 84,6 | - 0,3 | 6,9 | 63,9 |
| 13*) | 83,9 | - 0,1 | 7,4 | 61,7 |
| 14 | 85,1 | -0,4 | 6,3 | 66,2 |
| 15 | 85,2 | - 0,5 | 6,1 | 66,9 |
| *) Vergleichsbeispiele | | | | |

**Patentansprüche**

1. Verfahren zur Herstellung wasserabsorbierender Polymerpartikel durch Polymerisation einer Monomerlösung oder -suspension, enthaltend

   a) mindestens ein ethylenisch ungesättigtes, säuregruppentragendes Monomer, das zumindest teilweise neutralisiert sein kann,
   b) mindestens einen Vernetzer,
   c) mindestens einen Initiator,
   d) optional ein oder mehrere mit den unter a) genannten Monomeren copolymerisierbare ethylenisch ungesättigte Monomere und
   e) optional ein oder mehrere wasserlösliche Polymere,

   umfassend die Schritte Polymerisation der Monomerlösung zu einem Polymergel i), optional Zerkleinerung des erhaltenen Polymergels ii),Trocknung des Polymergels iii), Mahlung und Klassierung des getrockneten Polymergels zu Polymerpartikeln iv), und optional thermischer Oberflächennachvernetzung der klassierten Polymerpartikel v), **dadurch gekennzeichnet, dass** vor, während oder nach einem der Schritte i) bis v) getrennt oder zusammen

   - mindestens eine anorganische Phosphorsäure und/oder deren Salz und
   - mindestens eine organische 2-Hydroxysäure und/oder deren Salz,

   zugesetzt wird, wobei der Phosphor in der anorganischen Phosphorsäure eine Oxidationszahl von weniger als +V und die organische 2-Hydroxysäure keine ethylenisch ungesättigten Gruppen aufweist, und dass die organische 2-Hydroxysäure und/oder deren Salz vor Schritt i) zugesetzt wird.

**2.** Verfahren gemäß Anspruch 1, **dadurch gekennzeichnet, dass** die anorganische Phosphorsäure und/oder deren Salz nach Schritt i) und vor Schritt iii) zugesetzt wird.

**3.** Verfahren gemäß Anspruch 1 oder 2, **dadurch gekennzeichnet, dass** der mindestens eine Initiator c) ein Peroxid ist und in Schritt i) weniger als 0,1 Gew.-% eines Reduktionsmittels, bezogen auf die wasserabsorbierenden Polymerpartikel, zugesetzt wird.

**4.** Verfahren gemäß einem der Ansprüche 1 bis 3, **dadurch gekennzeichnet, dass** die anorganische Phosphorsäure unterphosphorige Säure und/oder phosphorige Säure ist.

**5.** Verfahren gemäß einem der Ansprüche 1 bis 4, **dadurch gekennzeichnet, dass** die organische 2-Hydroxysäure 2-Hydroxy-2-sulfonatoessigsäure, eine 2-Hydroxy-2-phosphonoessigsäure und/oder Hydroxyethyliden-1,1'-diphosphonsäure ist.

**6.** Verfahren gemäß einem der Ansprüche 1 bis 5, **dadurch gekennzeichnet, dass** von 0,001 bis 5 Gew.-% der anorganischen Phosphorsäure und/oder deren Salz, bezogen auf die wasserabsorbierenden Polymerpartikel, zugesetzt wird.

**7.** Verfahren gemäß einem der Ansprüche 1 bis 6, **dadurch gekennzeichnet, dass** von 0,001 bis 5 Gew.-% der organischen 2-Hydroxysäure und/oder deren Salz, bezogen auf die wasserabsorbierenden Polymerpartikel, zugesetzt wird.

**8.** Wasserabsorbierende Polymerpartikel, erhältlich nach einem Verfahren der Ansprüche 1 bis 7.

**Claims**

**1.** A process for producing water-absorbing polymer particles by polymerizing a monomer solution or suspension comprising

a) at least one ethylenically unsaturated monomer which bears acid groups and may be at least partly neutralized,
b) at least one crosslinker,
c) at least one initiator,
d) optionally one or more ethylenically unsaturated monomers copolymerizable with the monomers mentioned under a) and
e) optionally one or more water-soluble polymers,

comprising the steps of polymerizing the monomer solution to give a polymer gel i), optionally comminuting the resulting polymer gel ii), drying the polymer gel iii), grinding and classifying the dried polymer gel to polymer particles iv), and optionally thermally surface postcrosslinking the classified polymer particles v), which comprises adding, before, during or after one of steps i) to v), separately or together,

- at least one inorganic phosphoric acid and/or salt thereof and
- at least one organic 2-hydroxy acid and/or salt thereof,

where the phosphorus in the inorganic phosphoric acid has an oxidation number of less than +V and the organic 2-hydroxy acid does not have any ethylenically unsaturated groups, and adding the organic 2-hydroxy acid and/or salt thereof before step i).

**2.** The process according to claim 1, wherein the inorganic phosphoric acid and/or salt thereof is added after step i) and before step iii).

**3.** The process according to claim 1 or 2, wherein the at least one initiator c) is a peroxide, and less than 0.1% by weight of a reducing agent, based on the water-absorbing polymer particles, is added in step i).

4. The process according to any of claims 1 to 3, wherein the inorganic phosphoric acid is hypophosphorous acid and/or phosphorous acid.

5. The process according to any of claims 1 to 4, wherein the organic 2-hydroxy acid is 2-hydroxy-2-sulfonatoacetic acid, a 2-hydroxy-2-phosphonoacetic acid and/or hydroxyethylidene-1,1'-diphosphonic acid.

6. The process according to any of claims 1 to 5, wherein from 0.001 to 5% by weight of the inorganic phosphoric acid and/or salt thereof, based on the water-absorbing polymer particles, is added.

7. The process according to any of claims 1 to 6, wherein from 0.001 to 5% by weight of the organic 2-hydroxy acid and/or salt thereof, based on the water-absorbing polymer particles, is added.

8. Water-absorbing polymer particles obtainable by a process according to any of claims 1 to 7.

**Revendications**

1. Procédé de fabrication de particules polymères absorbant l'eau par polymérisation d'une solution ou suspension de monomères contenant

   a) au moins un monomère éthyléniquement insaturé, portant des groupes acide, qui peut être au moins partiellement neutralisé,
   b) au moins un agent de réticulation,
   c) au moins un initiateur,
   d) éventuellement un ou plusieurs monomères éthyléniquement insaturés copolymérisables avec les monomères mentionnés en a), et
   e) éventuellement un ou plusieurs polymères solubles dans l'eau,

   comprenant les étapes suivantes : la polymérisation de la solution de monomères en un gel polymère i), éventuellement le concassage du gel polymère obtenu il), le séchage du gel polymère iii), le broyage et la classification du gel polymère séché en particules polymères iv), et éventuellement la post-réticulation de surface thermique des particules polymères classées v), **caractérisé en ce qu'**avant, pendant ou après l'une quelconque des étapes i) à v), séparément ou ensemble,

   - au moins un acide phosphorique inorganique et/ou son sel et
   - au moins un 2-hydroxy-acide organique et/ou son sel,

   sont ajoutés, le phosphore dans l'acide phosphorique inorganique présentant un indice d'oxydation inférieur à +V et le 2-hydrox-acide organique ne comprenant pas de groupes éthyléniquement insaturés, et **en ce que** le 2-hydroxy-acide organique et/ou son sel sont ajoutés avant l'étape i).

2. Procédé selon la revendication 1, **caractérisé en ce que** l'acide phosphorique inorganique et/ou son sel sont ajoutés après l'étape i) et avant l'étape iii).

3. Procédé selon la revendication 1 ou 2, **caractérisé en ce que** le ou les initiateurs c) sont un peroxyde et, à l'étape i), moins de 0,1 % en poids d'un réducteur, par rapport aux particules polymères absorbant l'eau, est ajouté.

4. Procédé selon l'une quelconque des revendications 1 à 3, **caractérisé en ce que** l'acide phosphorique inorganique est l'acide hypophosphoreux et/ou l'acide phosphoreux.

5. Procédé selon l'une quelconque des revendications 1 à 4, **caractérisé en ce que** le 2-hydroxy-acide organique est un acide 2-hydroxy-2-sulfonatoacétique, un acide 2-hydroxy-2-phosphonoacétique et/ou un acide hydroxyéthylidène-1,1'-diphosphonique.

6. Procédé selon l'une quelconque des revendications 1 à 5, **caractérisé en ce que** 0,001 à 5 % en poids de l'acide phosphorique inorganique et/ou son sel, par rapport aux particules polymères absorbant l'eau, est ajouté.

7. Procédé selon l'une quelconque des revendications 1 à 6, **caractérisé en ce que** 0,001 à 5 % en poids du 2-hydroxy-acide organique et/ou son sel, par rapport aux particules polymères absorbant l'eau, est ajouté.

8. Particules polymères absorbant l'eau, pouvant être obtenues par un procédé selon les revendications 1 à 7.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0055245 A1
- WO 2006058682 A1
- WO 2004084962 A1
- WO 2008092842 A1
- WO 2008092843 A1
- WO 2009060062 A1
- WO 03014172 A2
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2

- WO 2001038402 A1
- DE 3825366 A1
- US 6241928 B
- WO 2008040715 A2
- WO 2008052971 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1
- EP 0640330 A1
- US 200502567575 A

**In der Beschreibung aufgeführte Nicht-Patentliteratur**

- **F.L. BUCHHOLZ ; A.T. GRAHAM.** Modern Super-absorbent Polymer Technology. Wiley-VCH, 1998, 71-103

- *Hunterlab,* 1996, vol. 8 (7), 1-4